# EUROPEAN PATENT APPLICATION

(11) **EP 4 528 259 A1**
(43) Date of publication of application: **26.03.2025**
(21) Application number: 23807010.6
(22) Date of filing: 17.05.2023
(51) Int. Cl.: G01N 27/327, G01B 5/00

(54) **BIOSENSOR AND MANUFACTURING METHOD THEREFOR**

(30) Priority: 18.05.2022 CN 202210547593
(71) Applicant: Leadway (HK) Limited, Sheung Wan, Hong Kong (CN)
(72) Inventor: JI, Xubo, Hangzhou, Zhejiang 310030 (CN); LEI, Tianran, Hangzhou, Zhejiang 310030 (CN); WANG, Yi, Hangzhou, Zhejiang 310030 (CN); WU, Mengqun, Hangzhou, Zhejiang 310030 (CN); GUO, Tao, Hangzhou, Zhejiang 310030 (CN); ZHANG, Li, Hangzhou, Zhejiang 310030 (CN)
(74) Representative: Schiweck Weinzierl Koch Patentanwälte Partnerschaft mbB
(86) International application number: PCT/CN2023/094908
(87) International publication number: WO 2023/222059

(57) **Abstract**

The present invention provides a biosensor and method. The biosensor includes an insulating substrate, a conductive layer disposed on the insulating substrate, a sample injection port, and a fluid channel for entrance of a test sample. Engraved lines and electrodes divided by the engraved lines are distributed on the conductive layer. A reagent layer is disposed on part or all of the electrodes located in the region of the fluid channel, a transverse engraved line is disposed on the other side opposite to the sample injection port, and at least one end of the transverse engraved line extends towards, but does not intersect with a longitudinal side edge of the biosensor. Through the arrangement of the engraved line at the contact end of the biosensor described in the present invention, the quality inspection process of the production process can be effectively simplified, and the pass rate of the biosensor can be improved.

## Description

### Field of the Invention

The present invention relates the biosensor technology in the field of biomedical testing, in particular to a biosensor and a preparation method thereof.

### Background of the Invention

Electrochemical sensors for detecting the content of an analyte in a sample and their matched detectors have been widely used in the daily monitoring of diseases. For example, diabetic patients commonly use electrochemical sensors to monitor daily glucose concentrations in the blood.

The basic structure of such electrochemical biosensors includes an electrode system disposed on an insulating substrate, the electrode system includes multiple types of electrodes such as a working electrode and a counter electrode, and a test reagent that reacts with the analyte covers the corresponding electrode. A sample interlayer having a groove is located on the electrodes, a cover plate with an air hole covers the sample interlayer, the insulating substrate, the interlayer and the cover plate form a sample injection channel, and the other end of the electrode system is provided with a contact for contacting a contact pin of a detector, wherein the end of the sensor having the sample injection channel is referred to as a sample injection end, and the end of the sensor contacting the contact pin of the detector is referred to as a contact end. The sample flowing into the sample injection channel reacts with the test reagent on the electrodes to generate an electrical signal from which the detector derives the test result.

The screen printing technology is utilized to print conductive materials on the insulating substrate to form electrodes, electrode connecting contacts and leads, which is a method commonly used for preparing electrochemical sensors at present. When electrochemical sensors are manufactured using the screen printing method, large batch-to-batch variations exist in aspects of certain parameters from batch to batch. In order to eliminate the extent to which the batch-to-batch variations affect the test results, the problem of batch-to-batch variations is generally solved by inserting a calibration chip into the detector to correct the test results. However, inserting a calibration chip not only increases the operation steps of an operator, but also leads to inaccurate test results if the operator forgets to insert or incorrectly inserts the calibration chip. Some researchers also adopted, for example, the method in Chinese Patent ZL201210095096.5 to correct the batch-to-batch variations by setting calibration messages on the sensor.

In order to solve the problem of cumbersome testing steps or complicated preparation process mentioned above, Chinese Patent ZL00803756.6 provides a method for forming a thin film electrode, i.e., a thin film-like conductor layer is uniformly spread on an insulating substrate in advance, and then the electrode is formed by a laser ablation method. This method has relatively high manufacturing precision and basically can ensure no batch-to-batch variations between products produced in different batches, or that the presence of batch-to-batch variations will not affect the test results in the case where no correction is required.

For such a sensor with electrodes formed by the laser ablation method, in the production process of cutting a large-layout semi-finished large card consisting of a plurality of sensor basic units to obtain finished sensors, it is necessary to ensure that the electrode will not be damaged thereon due to fluctuations in the cutting process to result in scrapping of the finished products or the aesthetics thereof will be not affected as the sample injection channel is not in a position close to the central axis of the sensor resulted from fluctuations in the cutting process. In order to solve the above problems, Chinese Patent ZL00803756.6 also proposes a method of fixing the electrode area to ensure that the electrode will not be scrapped due to fluctuations in the cutting assembly process, i.e., slits extending to the end edges at the sample contacting end and the contact contacting end, respectively, are disposed on the left and right side edges of the electrode of the sensor. The method is low in manufacturing difficulty, and the product performance can be ensured as long as the two slits are not touched during cutting.

In the solution recorded in Chinese Patent ZL00803756.6, the slits extend to the end edges of the sample contacting end and the contact end, respectively, and the slit size is longer, resulting in a longer laser travel line in the laser etching process. In the actual production process, the time for etching different materials will grow to different extents, for example, for laser etched products with limited production capacity, such as carbon films and conductive glass, the production time will be greatly increased, limiting the production capacity. Therefore, the present invention carries out research and development for the above problems, with the objective of providing a simpler design which can not only ensure the performance but also improve the production efficiency and capacity.

### Summary of the Invention

The present invention is completed based on the above problems in the prior art, and has an objective of providing a biosensor provided with an engraved line for identifying cutting deviation in the aspect of design. By observing the engraved line for identifying cutting deviation, it can be easy and convenient to screen out the scrapped products due to damage to electrodes caused by cutting deviation in the cutting process of the biosensor, or the defective products with sample injection grooves not within a reasonable range close to the central axis of the sensors.

The biosensor in the present invention comprises an insulating substrate and a conductive layer disposed on the insulating substrate, electrodes being formed on the conductive layer and divided by engraved lines. A test reagent is allocated to the electrodes. The biosensor is provided with a fluid channel for entrance of the solution of a substance to be tested, and the fluid channel is formed by the conductive layer for the electrodes, an open groove of an interlayer, and an upper cover superposed on each other. The other end of the electrode system is provided with a region that can touch a contact of a detector, a part of the region being exposed and not covered by materials such as a color layer. The end of the sensor having the sample injection channel is referred to as a sample injection end, and the end of the sensor contacting the contact of the detector is referred to as a contact end. The electrode extends from the sample contacting end to the contact end of the sensor and conducts the electrical signal generated by the reaction of the substance to be tested with the reagent to the contact end of the sensor, and then the test result is fed back to the user via an instrument. In order to prevent damage to the electrodes caused by cutting deviation of semi-finished products of the biosensor or poor appearance of the sensor, an engraved line is disposed on the electrodes, which is an engraved line for identifying cutting deviation and can help a quality inspector efficiently determine whether the cutting deviation meets requirements. When the engraved line for identifying cutting deviation can be obviously observed in the product after the cutting assembly or when the engraved line for identifying cutting deviation is still complete, the product can be determined as a qualified product. Because of the design of the engraved line for identifying cutting deviation, it is possible to easily and accurately screen out scrapped products due to damage to electrodes caused by cutting errors or defective products with poor appearance.

A first aspect of the present invention is that an engraved line for identifying cutting deviation is disposed at the contact end of the biosensor, and extends towards, but does not intersect with two side edges of the biosensor. Whether the cut biosensor meets requirements is quickly determined by observing the engraved line for identifying cutting deviation, so as to remove scrapped and defective products incorrectly cut in time, which ensures the stability of the finished sensor.

A second aspect of the present invention is that engraved lines for identifying cutting deviation are disposed on both sides of the biosensor, are adjacent to the side edges of the sensor, but do not intersect with any of the engraved lines at the sample contacting end and the instrument contact end of the biosensor. When the semi-finished large card of the biosensors is cut to obtain finished sensors, if an exposed engraved line for identifying cutting deviation can be seen on both sides, the sensor will be recognized as a finished product cut qualifiedly; otherwise, the sensor is a scraped product or a defective product, which can be quickly identified and removed.

For the presence of the above engraved line for identifying cutting deviation, it may also be such a case that one end thereof extends towards the sample contacting end and intersects the engraved line at the sample contacting end, while the other end thereof does not intersect the engraved line at the contact end, and such a design reduces the jumping of the engraved line in the laser etching process. That is to say, the engraved line for identifying cutting deviation may be obtained by consecutively etching the engraved line at the sample contacting end, which reduces the jumping of the engraved line and the idle waiting time of laser, and further improves the efficiency of laser etching. Based on the same consideration, the end of the engraved line that does not intersect the engraved line at the contact end can extend towards the side edge of the sensor to obtain a second engraved line, and intersect the side edge of the sensor, and these combined engraved lines for identifying cutting deviation is "π"-shaped in the biosensor. From the perspective of the large electrode card obtained after laser etching, such design can connect the engraved lines of adjacent sensors sequentially, so as to realize uninterrupted etching of the laser engraved lines, thus further enhancing the efficiency of laser etching and improving the production capacity.

A third aspect of the present invention is a further optimization made on the basis of the above second aspect by comprehensively considering the influence of a double-sided adhesive tape and a color layer affixed to the semi-finished large card on the engraved line for identifying cutting deviation and the influence of the length of the engraved line for identifying cutting deviation on the laser etching efficiency. More specifically, most of the engraved line for identifying cutting deviation in the biosensor mentioned in the second aspect of the present invention is covered under the double-sided adhesive tape and the color layer, and is relatively long, so the required laser etching time is correspondingly relatively long; and this engraved line for identifying cutting deviation is susceptible to influence of the upward and downward deviation of the affixed positions of the double-sided adhesive tape and the color layer to result in wrong determination. Based on the above situation, the biosensor of the third aspect described in the present invention is provided with engraved lines for identifying cutting deviation on both sides thereof, and one end of the engraved line intersects a transverse engraved line at the contact end of the biosensor, and the transverse engraved line at the contact end of the biosensor may or may not intersect the two side edges of the sensor. More preferably, the other end of the engraved line for identifying cutting deviation located on both sides of the biosensor does not extend towards the sample contacting end and does not intersect any of the engraved lines at the sample contacting end, and a third engraved line at this end flexes to extend towards both side edges of the biosensor and intersect the side edges of the sensor. Thus, the engraved line composed of the third engraved line, the engraved lines for identifying cutting deviation on both side edges and the transverse engraved line at the contact end is π-shaped in the sensor. The π-shaped engraved line can connect the engraved lines of adjacent sensors sequentially, so as to realize uninterrupted etching of the laser engraved lines, thus further enhancing the efficiency of laser etching and improving the production capacity.

The above aspects are further illustrated.

The present invention provides a biosensor, comprising an insulating substrate, a sample injection port, a fluid channel for entrance of a test sample, and a conductive layer disposed on the insulating substrate, engraved lines and electrodes divided by the engraved lines being distributed on the conductive layer, a reagent layer being disposed on part or all of the electrodes located in the region of the fluid channel, wherein a transverse engraved line 42 is disposed at a contact end on the other side opposite to the sample injection port of the biosensor, and at least one end of the transverse engraved line 42 extends towards, but does not intersect, the longitudinal side edge of the biosensor.

Further, no engraved line exists between the end of the transverse engraved line 42 extending towards the longitudinal side edge of the biosensor and the longitudinal side edge.

Further, the end of the transverse engraved line 42 close to the longitudinal side edge of the biosensor is connected with a vertical engraved line 31a or 32a, and the vertical engraved line is parallel to or at a certain angle to the longitudinal side edge of the biosensor.

Further, no engraved line exists between the vertical engraved line 31a or 32a and the longitudinal side edge of the biosensor on the same side.

Further, two ends of the transverse engraved line 42 extend towards, but do not intersect with two longitudinal side edges of the biosensor.

Further, two ends of the transverse engraved line 42 are connected with a vertical engraved line 31a or 32a, and the vertical engraved line is parallel to or at a certain angle to the longitudinal side edge of the biosensor.

Further, the vertical engraved line 31a or 32a intersects the side edge of the biosensor. Still further, when two or more biosensors are arranged together side by side, adjacent vertical engraved lines of two adjacent biosensors, which intersect the side edge, can be connected to each other.

Further, the other end of the vertical engraved line 31a or 32a opposite to the end connected with the transverse engraved line 42 is connected with another transverse engraved line, and the other transverse engraved line 31b or 32b intersects a side edge of the biosensor.

Further, the transverse engraved line 42, the vertical engraved line 31a or 32a and the other transverse engraved line 31b or 32b are connected to each other to be in a π shape. Still further, when two or more biosensors are arranged together side by side, the other transverse engraved lines of two adjacent biosensors can be connected to each other.

Further, the biosensor comprises an engraved line, and when two or more biosensors are arranged together side by side, adjacent engraved lines of two adjacent biosensors can be connected to each other. Still further, when two or more biosensors are arranged together side by side, engraved lines of two adjacent biosensors at the contact ends can be connected to each other.

Further, when two or more biosensors are arranged together side by side, engraved line repeating units connected to each other and having the same shape are formed at the contact end.

Further, the end of the transverse engraved line 42 extending towards the longitudinal side edge of the biosensor is observable.

Further, the transverse engraved line, the vertical engraved line and the other vertical engraved line are observable.

Further, the transverse engraved line located at the contact end stretches across the electrodes or the region that the electrodes extend towards the contact end.

Further, the biosensor comprises a working electrode and a counter electrode.

Further, the transverse engraved line or the vertical engraved line is set in such a way that when the transverse engraved line or the vertical engraved line on the biosensor meets preset requirements, the electrode at the sample contacting end of the biosensor is complete, and/or the central axis of a sample injection groove is located on the central axis of the biosensor or deviates to an extent within a preset range.

The engraved lines described in the present invention are not only straight lines, but also can be arcs, wavy lines and other curves. Similarly, the engraved lines for identifying cutting deviation are not only straight lines, but also can be arcs, wavy lines and other curves. Further, the transverse engraved line does not mean that it needs to be parallel to the two end edges of the sample end and contact end of the biosensor, and the transverse engraved line may be parallel to the end edges or at an angle to the end edges. The vertical engraved line also does not mean that it needs to be parallel to two side edges of the biosensor, and the vertical engraved line may be parallel to the side edges or at an angle to the side edges. The transverse engraved line or the vertical engraved line can be a straight line, a wavy line or in other shape.

The present invention also provides a method for preparing a biosensor, comprising the following steps: etching electrodes and the above engraved lines on an insulating substrate having a conductive layer according to a pre-designed electrode pattern, so as to form a large electrode card having a plurality of sensor base units; adding a prepared reagent to corresponding electrodes requiring addition of the reagent; affixing an interlayer to the sample contacting end of each sensor base unit; arranging an upper cover on the interlayer to obtain a semi-finished large card; cutting the semi-finished large card along preset cutting lines using a cutter to obtain finished biosensors; and removing the biosensors that do not completely render the engraved lines and are unqualifiedly cut, whereas retaining the biosensors that are qualifiedly cut.

The present invention also provides a method for determining whether a biosensor is cut correctly when being manufactured, comprising: setting an engraved line for identifying cutting deviation at a contact end on the other side of the biosensor opposite to the sample injection port, the engraved line for identifying cutting deviation comprising a transverse engraved line, at least one end of the transverse engraved line extending towards, but not intersecting with longitudinal side edges of the biosensor; and observing whether the engraved line for identifying cutting deviation meets pre-designed requirements, and if not, determining that the cutting is incorrect.

Further, the finished biosensor obtained after cutting is observed, and if a biosensor does not completely render the engraved line, it is determined that the cutting is incorrect.

The present invention also provides a biosensor, comprising an insulating substrate, a sample injection port, a fluid channel for entrance of a test sample, and a conductive layer disposed on the insulating substrate, engraved lines and electrodes divided by the engraved lines being distributed on the conductive layer, a reagent layer being disposed on part or all of the electrodes located in the region of the fluid channel, wherein vertical engraved lines 31a, 32a adjacent to two side edges of the biosensor are disposed on two sides of the biosensor, and at least one end of the vertical engraved line does not intersect the end edge of the biosensor.

Further, no engraved line exists between the vertical engraved line and the side edge of the biosensor.

Further, the two vertical engraved lines have the same line width.

Further, one end of each of the two vertical engraved lines extends towards the sample end of the biosensor and intersects the transverse engraved line at the sample end, respectively.

Further, one end of each of the two vertical engraved lines extends towards the contact end of the biosensor, respectively, and the endpoints of the extended ends intersect both ends of the same transverse engraved line at the contact end, respectively.

Further, at least a segment of the vertical engraved line adjacent to the two side edges of the biosensor is observable.

Further, the other end of the vertical engraved line not connected with the transverse engraved line is also connected with another transverse engraved line, and the other transverse engraved line extends towards and intersects the side edge of the biosensor. Further, when two biosensors are arranged together side by side, the engraved lines of two biosensors located at the contact end can be connected to each other.

Further, when two or more biosensors are arranged together side by side, engraved line repeating units connected to each other and having the same shape are formed at the contact end.

Further, the vertical engraved line is set in such a way that when the vertical engraved line adjacent to the two side edges of the biosensor can be seen completely, the electrode at the sample contacting end of the biosensor is complete, and/or the central axis of a sample injection groove is located on the central axis of the biosensor or deviates to an extent within a preset range.

The beneficial effects of the present invention mainly include: engraved lines 42, 31a, 31b, 32a, 32b, 31c, 32c, etc. for identifying cutting deviation are disposed on the biosensor. By observing the engraved lines for identifying cutting deviation, it can be easy and convenient to screen out the scrapped products due to damage to electrodes caused by cutting deviation in the cutting process of the biosensor, or the defective products with sample injection grooves not within a reasonable range close to the central axis of the sensors. The method is simple and convenient to operate, not only can ensure the product performance, but also can improve the product efficiency and capacity. The engraved line for identifying cutting deviation is a continuous uninterrupted engraved line on the biosensor, for example, π-shaped. Such design can connect the engraved lines of adjacent sensors sequentially, so as to realize uninterrupted etching of the laser engraved lines, thus further enhancing the efficiency of laser etching and improving the production capacity.

### Brief Description of the Drawings

Fig. 1 is an exploded view of a biosensor of Example 1.
Fig. 2 is a schematic diagram of the distribution of an electrode system and engraved lines on a conductive layer of the biosensor in Example 1.
Fig. 3 is a manufacturing flow of the biosensor.
Fig. 4 is a schematic diagram of multi-lined sensor base units A formed by laser engraving of the conductive layer of the biosensor in Example 1.
Fig. 5 is a schematic diagram of the single-lined sensor base units A formed after cutting by a cutter along the cutting line 50 as shown in Fig. 4.
Fig. 6 is a schematic diagram of the first type of biosensors in Example 1 arranged side by side before cutting.
Fig. 6-1 shows a single independent biosensor obtained after correct cutting of Fig. 6, and enlarged schematic diagrams thereof at L and R.
Fig. 6-2 shows a single independent unqualified biosensor obtained after incorrect cutting of FIG 6, and enlarged schematic diagrams thereof at L and R.
Fig. 6-3 is a schematic diagram of a second type of biosensors in Example 1 arranged side by side before not being cut.
Fig. 7 is a schematic diagram of the distribution of an electrode system and engraved lines of a first type of biosensors in Example 2, wherein a, b, and c represent biosensors formed after cutting at different positions, respectively.
Fig. 8 shows biosensors obtained after incorrect cutting according to the design solution of the first type of biosensors in Example 2, wherein a can be determined as a defective product and b can be determined as a scrapped product.
Fig. 9 is a schematic diagram of the distribution of an electrode system and engraved lines of a second type of biosensors in Example 2.
Fig. 10 is a schematic diagram of the distribution of an electrode system and engraved lines of a third type of biosensors in Example 2.
Fig.11 shows biosensors obtained after correct cutting according to the design solution of a first type of biosensors in Example 3, wherein a, b and c represent biosensors formed after cutting at different positions, respectively.
Fig. 12 shows biosensors obtained after incorrect cutting according to the design solution of the first type of biosensors in Example 3, wherein a can be determined as a defective product and b can be determined as a scrapped product.
Fig. 13 is a schematic diagram of sensors in other two solutions in Example 3, wherein the engraved lines 31a and 32a of the biosensor shown in a are arranged to be perpendicular to the engraved line 42, and the angle between each of the engraved lines 31a and 32a and the engraved line 42 of the biosensor shown in b is an obtuse angle.
Fig. 14 is a schematic diagram of an electrode system and engraved lines on a conductive layer corresponding to the biosensor further optimized in Example 3.
Fig. 15 shows biosensors obtained after incorrect cutting according to the design solution in Fig. 14, wherein a can be determined as a defective product and b can be determined as a scrapped product.
Fig. 16 is a schematic diagram of an uncut single-lined arrangement of the design solution shown in Fig. 14 in Example 3.
Fig. 17 is a schematic diagram of an electrode system and engraved lines on a conductive layer of one of the biosensors in Example 3.
Fig. 18 is a schematic diagram of the single-lined arrangement of the uncut biosensors in Fig. 17.
Fig. 19 is a schematic diagram of the arrangement of quality inspection contacts during the testing of a semi-finished product, wherein a is a schematic diagram in which six contacts for quality inspection are disposed, and b is a schematic diagram in which five contacts for quality inspection are disposed.

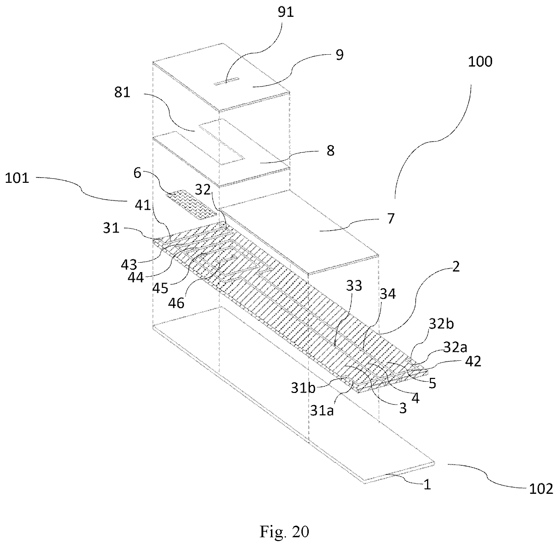

Fig. 20 is an exploded schematic diagram of the biosensor corresponding to Fig. 14.
Fig. 21 is a schematic diagram of the design of biosensors having six contacts for quality inspection.
Fig. 22 is a line graph of the biosensor test values versus the YSI test values of Example 3.

### Detailed Description of the Embodiments

### Example 1:

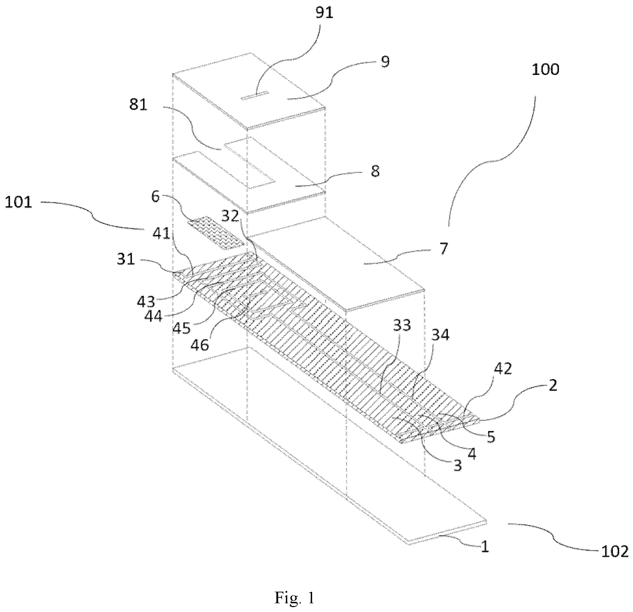

The biosensor 100 as shown in Fig. 1 includes an insulating substrate 1, a conductive layer 2 disposed on the substrate, and an electrode system formed on the conductive layer through division by engraved lines 31, 32, 33, 34, 41, 42, 43, 44, 45, 46. The electrode system includes a working electrode 5, a counter electrode 4 and a reference electrode 3, and the electrodes have a sample contacting end 101 contacting a sample and a contact end 102 contacting a contact of an analyzer. A reagent layer 6 is added to corresponding electrodes at the sample contacting end. An interlayer 8 with an open groove 81 covers the sample contacting end of the electrodes, and an upper cover 9 covers the interlayer 8, so that the conductive layer 2, the open groove 81 and the upper cover 9 are superposed together to form a fluid channel for entrance of a fluid under test. The electrodes and the reagent layer are in the channel. A sample injection port is formed on one side of the fluid channel. The upper cover 9 is provided with an air hole 91, which is located above the open groove and used for discharging air in the fluid channel after addition of the sample to be tested. A color layer 7 is printed with a color or identification system for identifying the sensor and is tightly adhered to the interlayer. Two side edges of the biosensor are 21 and 23, and the end edges of the sample end and contact end of the biosensor are 22 and 24.

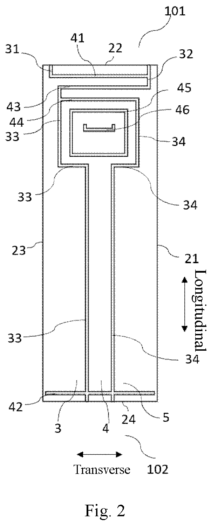

As shown in Fig. 2, it is a front view of the conductive layer 2 in Fig. 1, the engraved lines are insulating gaps left by the removal of a conductive material from the conductive layer 2. The engraved lines are not limited to gaps formed by means of engraving. The working electrode 5 is enclosed by the engraved lines 32, 33, 34, 42, 43, 44, the counter electrode 4 is enclosed by the engraved lines 33, 34, 42, 44, 45, and the reference electrode 3 is enclosed by the engraved lines 31, 32, 33, 41, 42, 43. In the preparation process of the biosensor, the engraved line 46 serves as a reference line for affixing of the interlayer, so as to ensure that the interlayer is affixed to a correct position.

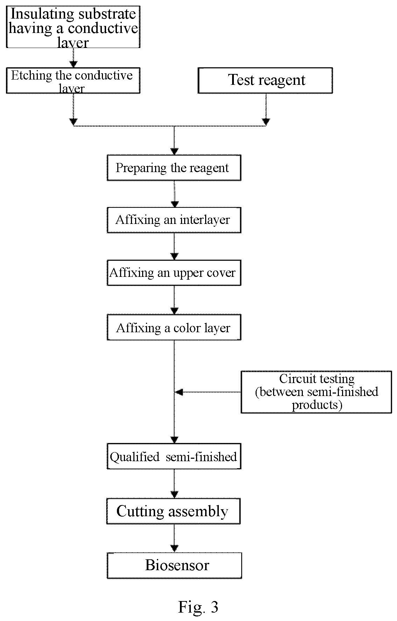

A method for manufacturing the biosensor shown in Fig. 1 is illustrated by taking Figs. 3, 4 and 5 as an example.

Step 1: laser etching of an electrode pattern: according to a pre-designed electrode pattern, on an original material having an insulating substrate and a conductive layer, etching the electrode pattern or other engraved lines with laser on the conductive layer, thus obtaining a large electrode card with a plurality of sensor base units A. The sensor base unit A includes engraved lines and electrodes formed through division by the engraved lines.

Step 2: addition of the reagent layer: preparing a test reagent, and adding the prepared reagent to corresponding electrodes requiring addition of the reagent.

Step 3: affixing the interlayer 8 to each sensor base unit A. Generally, the interlayer is placed at the sample contacting end of the biosensor.

Step 4: sticking the upper cover 9 to each interlayer 8 and rolling.

Step 5: after the upper cover 9 is affixed, affixing and rolling the color layer 7 on the upper cover to obtain a semi-finished large card.

Step 6: cutting: cutting the semi-finished large card by using a cutter along a preset cutting line to obtain finished biosensors.

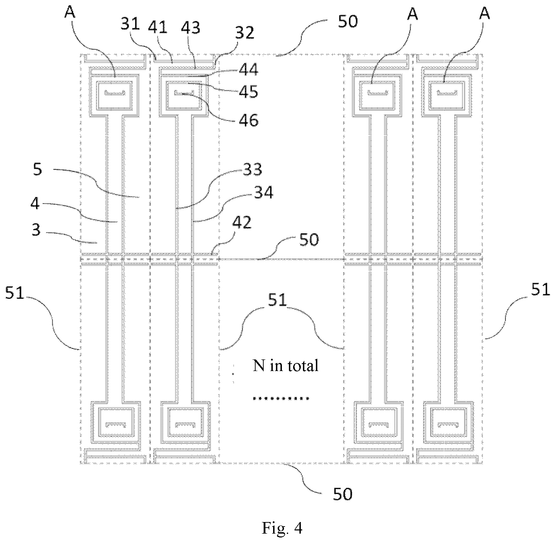

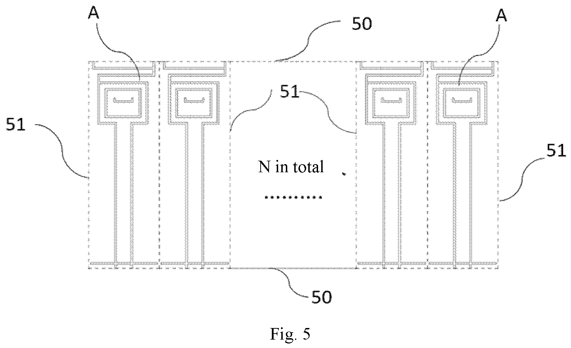

The manufacturing steps are specifically illustrated by taking Fig. 4 and Fig. 5 as an example. In step 1, the conductive layer 2 on the surface of the insulating substrate is etched using the laser etching technology to form a plurality of engraved lines 31 to 34 and engraved lines 41 to 46 on the conductive layer 2, and the conductive layer in these engraved lines is basically cleared away to expose the insulating substrate. Through division by the engraved lines, the required electrodes and other engraved lines with certain uses are formed on the conductive layer. After laser etching is completed, a large electrode card is obtained, including a plurality of sensor basic units A on an insulating substrate, which are arranged side by side and can be used to manufacture finished biosensors. Each sensor base unit A includes an insulating substrate, an electrode system formed after division by engraved lines, the engraved lines, and/or other blocks formed by etching. In this example, the electrode system includes the working electrode 5, the counter electrode 4, and the reference electrode 3. As shown in Fig. 4, two rows of sensor base units A arranged in sequence tail to tail are formed after etching (the contact ends of two sensor base units A are opposed to each other up and down), wherein N represents 0 to N; and "N in total" represents inclusion of N sensor base units A.

In step 1, i.e., laser etching of an electrode pattern, the etching travel line of the engraved lines within the conductive layer is controlled by software. Taking Fig. 4 as an example, the engraved lines 32, 43 and 33 are formed in such a way that they are obtained from continuous laser etching. The specific implementation of the path and direction and the like of the engraved lines can be artificially designed and controlled by software according to the needs of actual products, and is not limited to the above path. Suitable laser etching parameters are used for the engraved lines, e.g., the width of the engraved lines is within the range of 0.020mm to 0.200mm, and the width of the engraved lines used in this example is 0.080mm.

Fig. 4 is just one of the forms formed after step 1, and there may be other forms of arrangements as well, as long as the sensor base units A can be effectively cut in the back-end process. It is also possible to use Fig. 4 as a unit, and the large electrode card formed after etching is an arrangement of a plurality of units shown in Fig. 4, for example, if Fig. 4 is used as a repeating unit, N is 4 and the times of repetition is 2, the manufactured semi-finished product may have four rows and four columns.

The material of the insulating substrate is polyvinyl chloride, polyterephthalic acid, polyethylene terephthalate, macrogol ester, etc., with polyethylene terephthalate being preferred in this example.

The material of the conductive layer 2 is selected from, but not limited to conductive metals or conductive non-metals such as gold, silver, platinum, palladium, carbon, graphite, conductive glass, or a mixture thereof. The method for covering the conductive layer may use printing, coating, electroplating, sputtering, etc., and this example uses a conductive carbon layer (carbon film) formed in a coating manner, the thickness of the carbon layer is 1-30um, the thickness used in this example is about 8um, and the resistance of the conductive layer is 10 Ω/□ to 100 S2/o, preferably 30 Ω/□ in this example.

Some or all of the electrodes of the biosensor may be configured with a reagent layer. The reagent layer employs a specific enzyme to quantitatively or qualitatively analyze the measured target substance under the environment of a buffer system, generally containing the following components: enzyme, electronic mediator, high polymer, disintegrant, surfactant, stabilizer and buffer system. Depending on the needs of testing items, the enzyme is selected from one or more of glucose oxidase, glucose dehydrogenase, lactate oxidase, lactate dehydrogenase, urate oxidase, cholesterol oxidase or D-3-hydroxybutyrate oxidase, etc.; the electronic mediator is selected from one or more of ruthenium compounds, potassium ferricyanide, ferrocene, etc.; the buffer system is any one or more of sodium succinate, sodium citrate, piperazine buffer, propanesulfonic acid, PBS buffer or sodium fumarate; the disintegrant is any one or more of crosslinked PVP, sodium carboxymethyl starch, or crosslinked CCNa; the surfactant is any one or more of anionic surfactant, cationic surfactant, diatomic surfactant, or nonionic surfactant; and the stabilizer is one or more of maltitol, trehalose, BSA, or protein protective agent. The above components after preparation are stirred sufficiently, so that the components are fully dissolved and dispersed to form a homogeneous solution. In this example, the biosensor as shown in Fig. 1 includes a reagent layer 6.

In step 2 for preparing the reaction reagent, methods such as dispensing, screen printing, drop coating and Slot-Die coating are usually adopted to configure a reagent mixture having certain chemical components onto a specific electrode of the biosensor to form the reagent layer. It is preferred in this example that screen printing is used to configure a biochemical reagent on the specific electrode in specific pattern and position. The biochemical reagent in the specific pattern and position can react with the substance to be tested in the sample and generate a certain electrical signal, and the electrical signal is conducted to a test instrument and fed back to a user via a conductive material. The screen printing plate used in the screen printing generally employs polyester, nylon, stainless steel and other materials, and is generally of 250 meshes to 420 meshes, the used wire screen has a wire diameter of 27um to 120um generally, and bears a maximum tension of 22 to 38N/cm generally. It is preferred in this example that a 305-mesh nylon screen printing plate with a wire diameter of 34um is used, which can bear a maximum tension of 33N/cm.

The reagent with fixed pattern and position obtained via screen printing as described above comes into contact with the sample in the sample injection channel and reacts with the substance to be tested in the sample. The sample injection channel is formed between the open groove 81 and the upper cover 9, and the surface of the upper cover facing the sample injection channel is a hydrophilic layer material. Specifically, the material of the interlayer is typically a PET substrate coated with an acrylic resin system as an adhesive material. The thickness of the interlayer is typically 75um to 150um, and the width of the open groove is typically 0.7mm to 1.8mm. In this example, the thickness of the interlayer is preferably 100um, and the width of the hollow structure is preferably 1.2mm.

An air hole 91 is left in the upper cover 9 at the tail of the sample injection channel, and the air hole may be round, square, rectangular, linear, or in other shapes. It is preferred in this example that the air hole is rectangular. **In** this example, the hydrophilic material is preferably 9901P made by 3M. The air hole of the hydrophilic material can ensure that the original air in a chamber is smoothly discharged when a blood sample flows into the sample injection channel, so as to ensure that the sample can smoothly flow into the chamber.

The color layer 7 is generally a printable single-sided adhesive tape, with the product name generally printed on the surface, which plays a role of easily identifying the product and protecting the reagent strip from damage caused by scratching.

Step 5 is a step that can be omitted. For example, if the upper cover itself can be printed with product name, which can simultaneously serve a similar function as the color layer, step 5 is omitted. Alternatively, if the biosensor does not require the color layer, step 5 is omitted.

**In** the cutting step 6, the semi-finished large card is cut to obtain finished biosensors by using a cutter along a preset cutting line, specifically, using the cutter along the cutting lines at positions shown by transverse dotted lines 50 and longitudinal dotted lines 51 in Fig. 4. Suitable cutting methods include but not limited to hobbing, die punching, chopping, etc. Specifically, it is preferred in this example to use a hob to hob the semi-finished large card as shown in Fig. 4 along the transverse dotted line 50 into semi-finished strip-shaped sheets containing several sensor base units A as shown in Fig. 5, and then use the hob to cut the semi-finished strip-shaped sheets into finished biosensors along the vertical dotted line 51. **In** order to more visually illustrate the cutting position of the hob, when Fig. 4 and Fig. 5 are used to introduce the cutting process of step 6, the interlayer, the upper cover and the color layer are not shown in Fig. 4 and Fig. 5. The cutting process described in step 6 is one of the important steps in the assembly of the sensor, the effect of which directly affects the pass rate of the final finished sensors and the finished product effect of the sensors.

The dotted lines labeled in Fig. 4 and Fig. 5 do not exist in the actual product processing, the same below. After cutting, the positions of the dotted lines 50 and 51 are edge lines of the biosensor, which can also be referred to as edges, side edges or end edges.

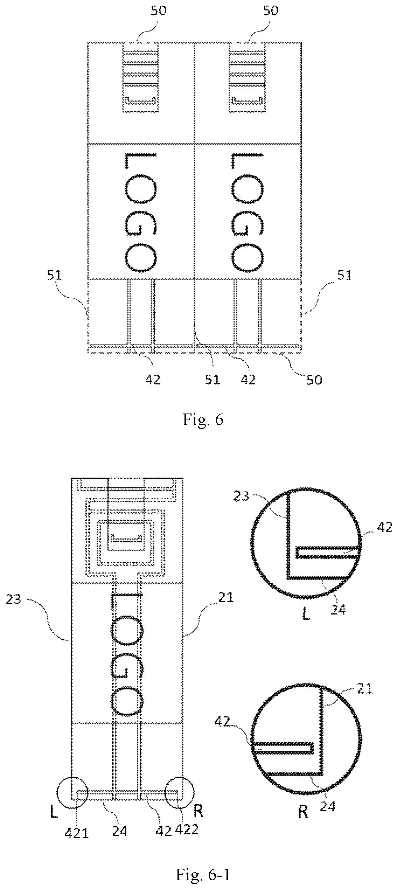

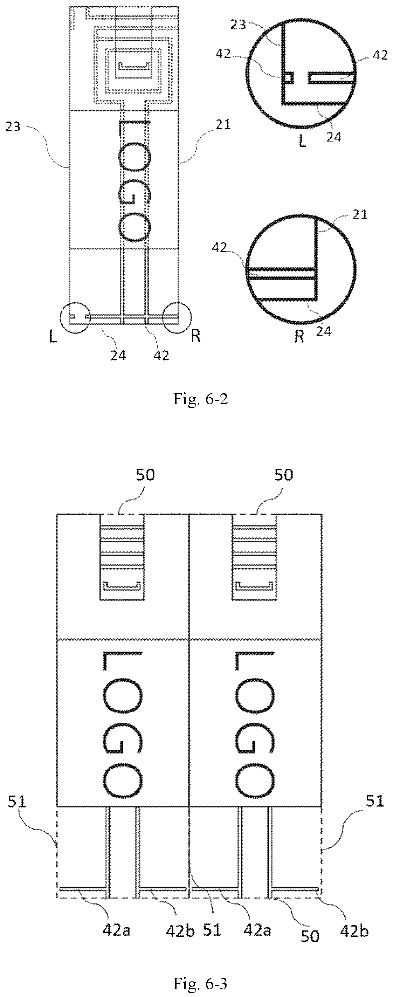

Figs. 6, 6-1 and 6-2 will be used below to specifically introduce how the cutting assembly process affects the finished product effect of the sensor and how to achieve identification and removal of scrapped or defective sensors after cutting assembly when preparing the biosensor in Figs. 1 and 2.

As shown in Figs. 1, 2 and 6, a transverse engraved line 42 transversing the electrode 3, the electrode 4 and the electrode 5 is disposed at the contact end of the biosensor, or the transverse engraved line 42 is disposed in the direction of the region that the electrodes extend towards the contact end. The engraved line 42 is observable, or at least two ends of the engraved line is observable, for example, the engraved line 42 is not wholly covered by the upper cover 9 or the color layer 7. The engraved line 42 does not intersect longitudinal side edges 23, 21, that is, the left and right endpoints 421 and 422 of the engraved line 42 are each at a certain distance from the longitudinal side edges 23, 21 of the sensor. In a preferred solution, no engraved line exists between an end of the transverse engraved line 42 extending towards a longitudinal side edge of the biosensor and this side edge.

Fig. 6 is a schematic diagram of the positions of two adjacent sensors waiting to be cut (in this example the color layer 7 represents a company logo with characters LOGO, and for ease of illustration, the reagent layer 6 is not embodied in the illustration for the time being, as is also the case in Fig. 6-1 and Fig. 6-2), and the cutter divides them into two independent biosensors along the preset cutting line 51.

Fig. 6-1 shows the cutter performs accurate cutting along the cutting lines 50 and 51, after which finished biosensors that meet the design requirements and are complete are obtained. The transverse engraved line 42 serves as an engraved line for identifying cutting deviation at the contact end of the sensor, the side edges 21, 23 of the sensor are obtained after cutting along the cutting line 51, and the end edges 22, 24 of the sensor are obtained after cutting along the cutting line 50. As can be seen from the enlarged schematic diagrams at L and R in Fig. 6-1, the engraved line 42 for identifying cutting deviation is observable at the contact end of the sensor, with left and right endpoints 421 and 422 thereof located on both sides of the sensor, respectively, and the engraved line 42 not intersecting the side edges of the sensor. Based on the above observed information, the quality inspector can consider that the obtained sensor is a complete finished sensor, at which point the electrode system of the finished sensor is cut along the cutting line 51 without cutting deviation, then the electrodes are not damaged, and the test performance of the sensor obtained by cutting is not affected; at the same time, the LOGO on the appearance thereof and the sample injection groove at the sample contacting end are both located close to the central axis of the finished sensor. For a user, at this point, the finished sensor has a relatively beautiful appearance and a visually comfortable user experience.

However, in the actual cutting assembly process, the cutter may cut at a preset position that deviates from the cutting line 51, and the appearance of the actually obtained sensor may change, and the quality inspector will determine the relative positions of the engraved line 42 for identifying cutting deviation and the side edges 21, 23 of the sensor to determine whether the cutting quality of the sensor obtained by cutting is good or poor. When the two endpoints of the engraved line 42 for identifying cutting deviation can be seen on the side of the contact end of the sensor, as shown in Fig. 6-2 and the enlarged schematic diagrams at L and R in this figure, two segments of the engraved line 42 for identifying cutting deviation that are not connected are seen on the left side of the contact end of the sensor, while on the right side, the engraved line for identifying cutting deviation intersects the side edge of the sensor, which indicates that the fluctuation when the cutter cuts along the cutting line 51 is out of the preset range, and the electrodes at the sample contacting end of the biosensor obtained at this point may be damaged by the cutting, which may have an impact on the test results. At the same time, in terms of the appearance of the biosensor, the position of the color layer with LOGO and the position of the sample injection groove at the sample contacting end are both out of the range of the central axis of the sensor, so that the sensor does not have a better appearance, and thus may result in a poorer user experience. Fig. 6-2 only shows the case where the cutting line deviates from one side, and the case where the cutting line deviates from the other side is similar, which will not be described in this example.

For ease of illustration, the engraved lines covered under the interlayer and the color layer in Figs. 6-1 and 6-2 are represented by dotted lines, while the exposed engraved lines that can be observed by the quality inspector are represented by solid lines. From the comparative analysis of the cutting situations in Figs. 6-1 and 6-2, it can be seen that by adding the engraved line for identifying cutting deviation, which does not intersect the side edges of the sensor, at the contact end of the sensor, it can help the quality inspector to carry out QC screening on the biosensors after cutting assembly to ensure the quality of the product. **In** the examples of Fig. 6, Fig. 6-1 and Fig. 6-2, when the endpoints of a complete engraved line for identifying cutting deviation can be seen on both sides of the contact end of the sensor after cutting, the obtained sensor can be considered to be an complete finished product; and when the endpoints of the engraved line for identifying cutting deviation can be seen on one side only, the sensor at this point is a scrapped or defective product, which should be removed by the quality inspector in time to prevent the sensor from flowing into the next production process. The scrapped and defective products after cutting can be simply and efficiently screened out through such method, thus effectively ensuring the stability and effectiveness of the delivered products.

Fig. 6-3 shows another design method of this example. Transverse engraved lines 42a and 42b are disposed in segments at the contact end of the biosensor. The engraved lines 42a and 42b serve as the engraved lines for identifying cutting deviation in the present design, the engraved line 42a is located on the electrode 3 or in the region that the electrode 3 extends towards the contact end, and the engraved line 42b is located on the electrode 5 or in the region that the electrode 5 extends towards the contact end. The engraved lines 42a and 42b are observable, or at least two ends of the engraved lines are observable, for example, the engraved lines 42a and 42b are not wholly covered by the upper cover 9 or the color layer 7. The left end of the engraved line 42a does not intersect the longitudinal side edge 23 of the sensor, and the right end of the engraved line 42b does not intersect the longitudinal side edge 21 of the sensor. The engraved lines 42a and 42b are disconnected, and neither of the two ends thereof intersects the side edges of the sensor. The quality inspector determines whether the sensor meets the quality requirements by observing the intersection of the engraved lines 42a and 42b with the side edges of the sensor, as well as the number of endpoints of the engraved line on a side of the sensor.

### Example 2

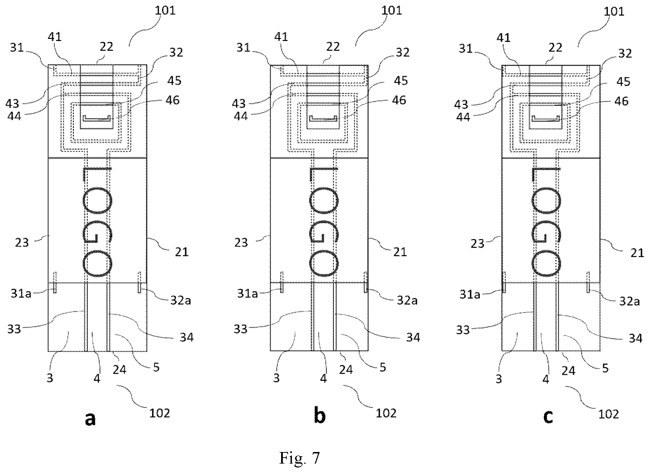

The biosensor 100 in the second aspect described in the present invention is stated by taking Fig. 7 as an example. The electrode system of the biosensor 100 is enclosed by insulated engraved lines formed by carrying out laser etching on the conductive layer covering the insulating substrate to remove some conductive layer. The sensor also includes a sample contacting end contacting a sample and a contact end contacting an analyzer. Specifically, at the sample contacting end, a fluid channel for entrance of the sample is composed of an interlayer with an open groove and an upper cover covering the interlayer and having an air hole, and part or all of the electrodes in the fluid channel are covered with a reagent layer.

The preparation process of biosensor 100 is basically consistent with that described in Example 1. As shown in Fig. 7, the engraved lines covered under the color layer and the interlayer are represented by dotted lines, the exposed engraved lines that can be seen are represented by solid lines. Vertical engraved lines 31a and 32a for identifying cutting deviation in the longitudinal direction are disposed on both sides of the sensor close to the side edges 23 and 21 of the sensor, the engraved lines 31a and 32a do not intersect any of the engraved lines at the sample contacting end and contact end of the sensor, and the engraved lines 31a and 32a are not covered at all or in part and are observable.

As shown in a in Fig. 7, it is a sensor obtained from cutting in strict accordance with cutting standards, complete cutting lines 31a and 32a can be seen on both sides of the sensor, at which point the cutting does not result in damage to any electrodes, the sample injection port and the LOGO on the color layer are also located close to the central axis of the sensor, and the sensor at this point has better performance and the most acceptable and comfortable appearance for the user. Appropriate and reasonable deviation exists in the cutting process, for example, two cases where the cutter cuts with offsets slightly to the left and slightly to the right, specifically as shown in b in Fig. 7 and in c in Fig. 7, and the standards for determining a qualified sensor product are still met in the states of b in Fig. 7 and c in Fig. 7. Although the cutting is offset to the left in b in Fig. 7 and to the right in c in Fig. 7, complete engraved lines 31a and 32a for identifying cutting deviation still can be seen on both sides of the contact end of the sensor, and such offsets are considered to be offsets within the expected range of the product design and are permitted.

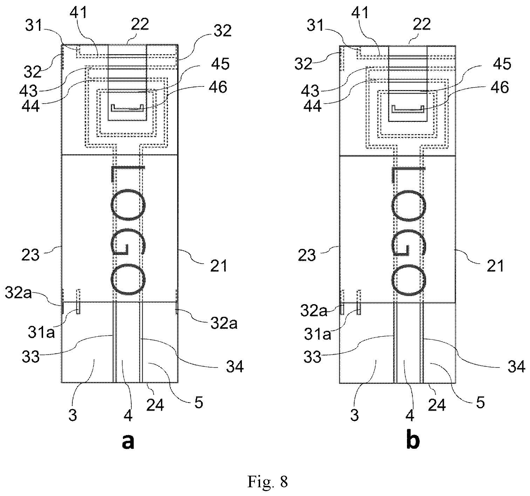

After cutting, the situations shown in a in Fig. 8 and in b in Fig. 8 occur to the biosensor, i.e., the two engraved lines 31a, 32a for identifying cutting deviation are seen at the same time on only one side of the sensor, or broken and incomplete engraved lines for identifying cutting deviation are seen on only one side, which indicates that the position cut by the cutter has been out of the expected range, and this sensor may be considered to be a scrapped or defective product.

The following will specifically illustrate how scrapped and defective biosensors are identified via the engraved lines 31a and 32a for identifying cutting deviation. As shown in Fig. 8, it is front schematic diagrams of two different sensors obtained after the semi-finished large card is subjected to the cutting process, wherein the engraved lines covered under the color layer and the interlayer are indicated by dotted lines, and the exposed engraved lines that can be seen are indicated by solid lines. As shown in a in Fig. 8, slight deviation exceeding the preset value occurs in the cutting process, so that the edge line 21 of the sensor obtained after cutting exactly passes through the engraved line 32a, and the engraved line 32a can be seen at positions next to both side edges of the sensor, but is incomplete and imperfect. At this point, although the cutter only cuts on the engraved line forming an electrode and the electrode itself is not damaged, the positions of the LOGO on the color layer of the sensor and the sample injection groove at the sample injection end have been out of the preset range of the central axis of the sensor, the performance of the sensor at this point is not affected, but the appearance thereof is unacceptable, so that the sensor can be considered to be a defective product. As shown in b in Fig. 8, large deviation occurs in the cutting process, the engraved lines 31a and 32a for identifying cutting deviation are located on the same side of the sensor but do not appear on the other side, which results in that the LOGO on the color layer of the sensor and the sample injection groove at the sample injection end have completely deviated from the preset range of the central axis of the sensor, meanwhile the electrode at the sample contacting end is also damaged, and the sensor at this point may be considered to be a scrapped sensor.

By providing engraved lines for identifying cutting deviation at positions on both sides of the sensor and adjacent to the side edges, it can help the quality inspector to quickly screen out the scrapped and defective products obtained after cutting. In the sensor obtained after cutting, when complete engraved lines for identifying cutting deviation can be seen on both sides of the sensor, the sensor is considered to be a good finished product; otherwise, the sensor is considered to be a scrapped or defective product. Compared to determining the transverse engraved line 42 for identifying cutting deviation disposed at the contact end in Example 1, such design has a better legibility, and at the same time improves the accuracy and ensures the stability of the products while ensuring the production efficiency of the product.

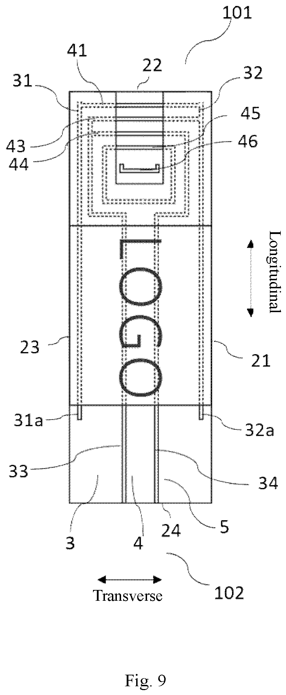

One end of each of the engraved lines for identifying cutting deviation located on both sides of the sensor and adjacent to the side edges of the sensor may also extend towards the sample contacting end of the sensor and intersect the engraved line at the sample contacting end. As shown in Fig. 9, the engraved lines 31a and 32a for identifying cutting deviation extend towards the sample contacting end, respectively, and intersect the engraved line 41 at the sample contacting end, respectively. The way of identifying cutting deviation of the sensor is substantially the same as that in the example in Fig. 7. In the design solution for the engraved line shown in Fig. 9, 31a and 32a can be connected via the engraved line 41 to achieve continuous cutting, thereby avoiding the idle jumping time of a laser when the engraved lines 31a and 32a for identifying cutting deviation are being cut, and improving the efficiency of laser etching.

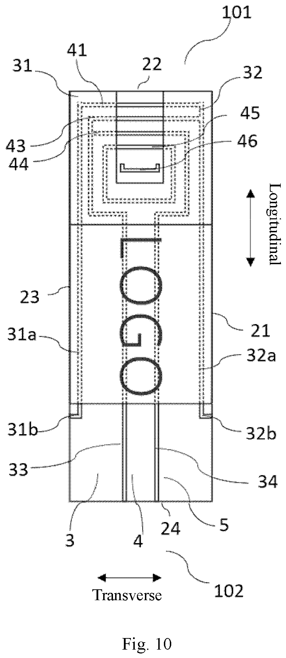

One end of each of the engraved lines for identifying cutting deviation located on both sides of the sensor and adjacent to the side edges of the sensor can also extend towards the sample contacting end of the sensor and intersect the engraved line at the sample contacting end, while the other end can also extend towards both side edges of the sensor to obtain second engraved lines and intersect the edge lines of the sensor. As shown in Fig. 10, one end of each of the engraved lines 31a and 32a for identifying cutting deviation extends towards the sample contacting end and intersects the engraved line 41, respectively, and the other end of each of the engraved lines 31a and 32a flexes and extends towards the two side edges of the sensor to obtain second engraved lines 31b and 32b and intersect the edge lines of the sensor, respectively. The engraved lines 31a and 32a for identifying cutting deviation and the engraved lines 31b, 32b and 41 intersecting them jointly form a "π"-shaped engraved line, which is in an inverted "π" shape as observed from the direction in Fig. 10. The way of determining whether the sensor undergoes cutting deviation using the engraved line for identifying cutting deviation as shown in Fig. 10 is basically the same as that in Fig. 7. The advantage of the electrode structure as shown in Fig. 10 over Fig. 9 is that in the electrode etching process of the sensor manufacturing process, the "π"-shaped engraved lines between consecutive adjacent sensors can be cut consecutively, which thus can reduce the time required for etching the large electrode card, thereby increasing the production efficiency and capacity.

### Example 3

The biosensor described in this example is a further optimization made on the basis of Example 2 by comprehensively considering the influence of an interlayer and a color layer affixed to the semi-finished large card on the engraved line for identifying cutting deviation and the influence of the length of the engraved line for identifying cutting deviation on the laser etching efficiency. A part of the engraved line for identifying cutting deviation of the biosensor mentioned in Example 2 is covered under the interlayer (also referred to as a double-sided adhesive tape) and the color layer, and the exposed part of the engraved line for identifying cutting deviation of the sensor subjected to cutting assembly, which can be seen by the quality inspector, is susceptible to the influence of the upward and downward deviation of the affixed positions of the double-sided adhesive tape and the color layer so as to be covered, thus increasing the determination difficulty of the quality inspector and increasing the risk of wrong determination. Based on the above situation, the biosensor of this example is provided with engraved lines for identifying cutting deviation on both sides of the sensor, and one end of each of the engraved lines intersects the transverse engraved line at the contact end of the biosensor, and the transverse engraved line at the contact end of the biosensor may or may not intersect the two side edges of the sensor.

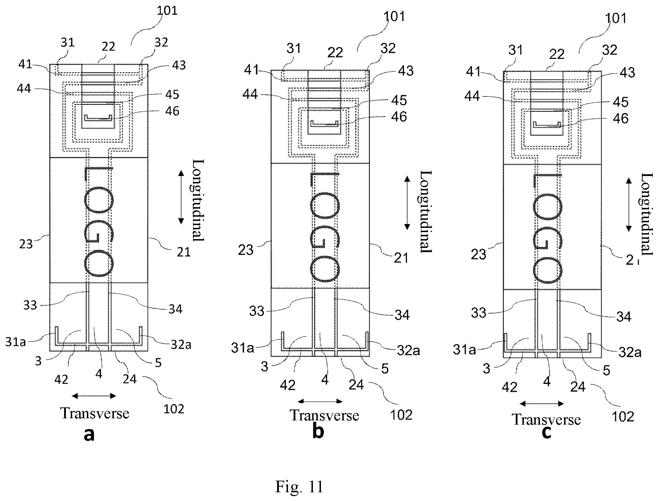

Fig. 11 shows a biosensor 100 in a third aspect of the present invention. The electrode system of the biosensor 100 is enclosed by insulated engraved lines formed by carrying out laser etching on the conductive layer covering the insulating substrate to remove some conductive layer. The sensor also includes a sample contacting end contacting a sample and a contact end contacting an analyzer. At the sample contacting end, a fluid channel for entrance of the sample is composed of an interlayer with an open groove and an upper cover covering the interlayer and having an air hole, and part or all of the electrodes in the fluid channel are covered with a reagent layer.

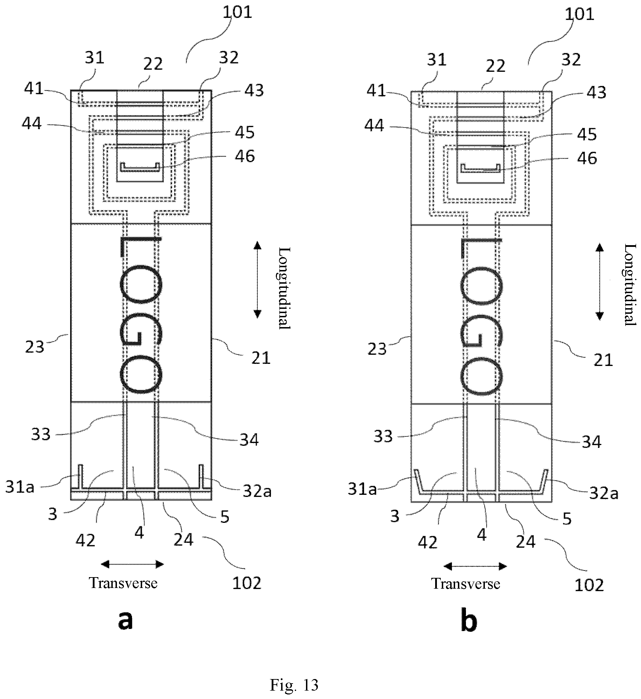

The preparation process of biosensor 100 is basically consistent with that described in Example 1. As shown in Fig. 11, it shows front schematic diagrams of sensors obtained after cutting assembly, wherein the engraved lines covered by the interlayer and the color layer are represented by dotted lines. A transverse engraved line 42 adjacent to the end edge 24 of the sensor and vertical engraved lines 31a and 32a for identifying cutting deviation located on both sides of the sensor and adjacent to the side edges of the sensor are designed at the contact end of the biosensor. The engraved line 42 does not intersect the side edges of the sensor, and the vertical engraved lines 31a and 32a do not intersect the side edges of the sensor either. The left and right ends of the engraved line 42 are connected to one end of the engraved line 31a and one end of the engraved line 32a, respectively, and the other end of each of the engraved lines 31a and 32a extends towards the sample contacting end of the sensor but is not connected to the end edge at the sample contacting end or the transverse engraved line close to the end edge. The lengths of the engraved lines 31a, 32a for identifying cutting deviation can be designed according to practical needs. At least the engraved lines at the intersections of the engraved line 42 with the engraved lines 31a and 32a are uncovered and observable. The end of the transverse engraved line 42 close to the longitudinal side edge of the biosensor is connected with the vertical engraved line 31a or 32a. In the solution described in Fig. 11, the vertical engraved lines 31a and 32a are parallel to the longitudinal side edges of the biosensor. In the solution described in Fig. 13, the vertical engraved lines 31a and 32a are at certain angles to the longitudinal side edges of the biosensor. No engraved line exists between the end of the transverse engraved line 42 extending towards a longitudinal side edge of the biosensor and this side edge.

As shown in a in Fig. 11, it is a sensor obtained after cutting according to cutting standards, complete engraved lines 31a, 32a and 42 can be seen, and both ends of the engraved line 42 do not intersect the side edges of the sensor. At this point, the cutting does not result in damage to the electrodes, the sample injection channel and the LOGO on the color layer are also located close to the central axis of the sensor, and the sensor at this point has better test performance and the most acceptable and comfortable appearance for the user. When appropriate and reasonable deviation exists in the cutting process, an offset cutting of the cutter to the left or to the right exists, as shown in b in Fig. 11 and c in Fig. 11. The standards for determining a qualified sensor product are still met in the states of b in Fig. 11 and c in Fig. 11. Although the cutting is offset to the left in b in Fig. 11 and to the right in c in Fig. 11, complete engraved lines 31a and 32a for identifying cutting deviation still can be seen on both sides of the contact end of the sensor, and such offsets are considered to be offsets within the expected design range of the product and are permitted.

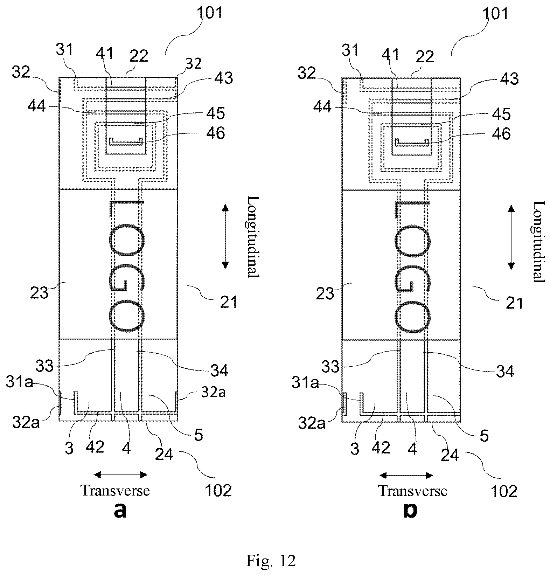

When the situations as shown by a in Fig. 12 and by b in Fig. 12 occur, i.e., the two engraved lines 31a, 32a for identifying cutting deviation are seen at the same time on only one side of the sensor, or broken and incomplete engraved lines 31a, 32a for identifying cutting deviation are seen on only one side, which indicates that the position cut by the cutter has been out of the expected range, and this sensor may be considered to be a scrapped or defective product.

The following will specifically illustrate how scrapped and defective biosensors are identified via the engraved lines 31a and 32a for identifying cutting deviation. As shown in Fig. 12, it is front schematic diagrams of two different sensors obtained after the semi-finished large card is subjected to the cutting process, wherein the engraved lines covered under the color layer and the interlayer are indicated by dotted lines, and the exposed engraved lines that can be seen are indicated by solid lines. As shown in a in Fig. 12, slight deviation exceeding the preset value occurs in the cutting process, so that the edge line 21 of the cut sensor obtained after cutting exactly passes through the engraved line 32a, and the engraved line 32a can be seen on both sides of the sensor, but is incomplete and imperfect. At this point, although the cutter only cuts on the engraved line forming the electrode and the electrode itself is not damaged, the positions of the LOGO on the color layer of the sensor and the sample injection groove at the sample injection end have been out of the preset range of the central axis of the sensor, the performance of the sensor at this point is not affected, but the appearance thereof is unacceptable, so that the sensor can be considered to be a defective product. As shown in b in Fig. 12, large deviation occurs in the cutting process, the engraved lines for identifying cutting deviation appear completely only on one side of the sensor but do not appear on the other side, which results in that the LOGO on the color layer of the sensor and the sample injection groove at the sample injection end have completely deviated from the theoretical range of the central axis of the sensor, meanwhile the electrodes at the sample contacting end are also damaged, and the sensor at this point may be considered to be a scrapped sensor.

Compared with the solutions in Fig. 9 and Fig. 10, in the solution shown in Fig. 11, the total length of the travel path of laser etching is shortened obviously, which can reduce the time spent in the laser etching process, so as to improve the efficiency and the production capacity. Moreover, the solution is not affected by the affixed positions of the interlayer and the color layer, and the situation that the engraved line for identification is covered by the interlayer and the color layer and thus is not observed will not occur. The relative positions of the engraved lines 31a, 32a and the side edges 21, 23 of the sensor are more intuitive and easier to identify and interpret than the relative positions of the endpoints of the engraved line 42 for identifying cutting deviation and the side edges 21, 23 of the sensor in Example 1, so that the quality inspector can identify the positions more simply and quickly, reducing the reading errors that may be caused by relying only on interpreting the positions of the endpoints of the engraved line 42, and improving the efficiency and accuracy of the production process.

In the above embodiment, the lengths of the engraved lines for identifying cutting deviation located on both side edges of the biosensor can be shortened or lengthened according to the actual needs. As shown in a in Fig. 13, the engraved line 42 located at the contact end can also extend towards and intersect the two side edges of the sensor. The engraved line 42 extending to and intersecting the side edges of the sensor can be consecutively etched from one side of the first sensor to the other side of the engraved line 42 of the Nth sensor in laser etching of a large card, thereby reducing the jumping of the laser etching path and the idle jumping time of the laser.

As shown in b in Fig. 13, the included angle between each of the engraved lines 31a and 32a and the engraved line 42 is an obtuse angle, and the quality inspector determines the schematic engraved lines 31a and 32a on the sensors produced after cutting assembly, and when complete schematic engraved lines 31a and 32a can be seen on the two sides of the sensor, that is, when we can see obtuse angles formed by the complete schematic engraved lines 31a and 32a for identifying cutting deviation and the engraved line 42, respectively, none of the engraved lines 42, 31a and 32a intersects the side edges of the sensor, then the sensor can be considered to be a qualified sensor.

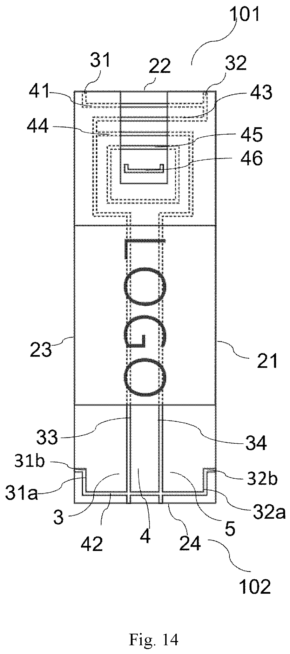

The solution of Fig. 14 is an improved design based on the solution of Fig. 11 of this example. The preparation process of the biosensor 100 as shown in Fig. 14 is kept consistent with those described in Example 1 and Example 2. The design of the engraved lines is basically the same as those in Fig. 11, with the difference that the carving lines 31a and 32a for identifying cutting deviation on the side edges flex and extend towards the two side edges of the sensor to obtain the engraved lines 31b and 32b and intersect the side edges of the sensor, respectively, and the engraved lines 31b and 32b can be parallel to the end edges or at a certain angle with the end edges. The continuous engraved line consisting of the engraved lines 31a, 31b, 32a, 32b and the engraved line 42 is in an inverted "π" shape.

As shown in Fig. 14, when complete engraved lines 31a, 32a for identifying cutting deviation are seen on the left and right sides of the sensor, it can be assumed that the cutting does not result in damage to any electrodes, and the sample injection channel and the LOGO on the color layer are also located in the preset range of the central axis of the sensor, and the sensor at this point has better performance and the most acceptable and comfortable appearance for the user.

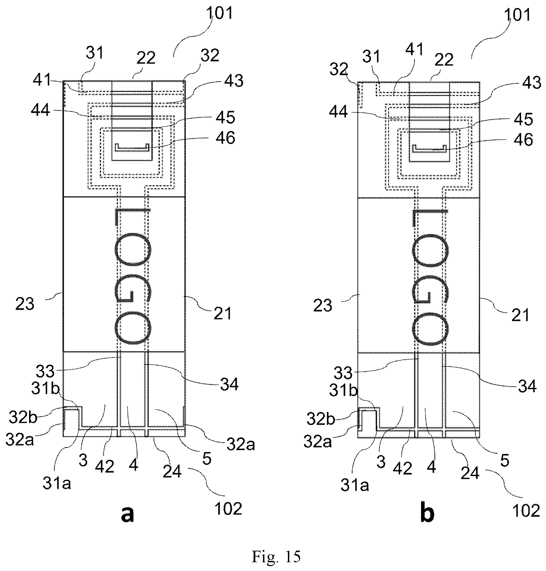

As shown in a in Fig. 15, slight deviation exceeding the preset value occurs in the cutting process, so that the edge line 21 of the sensor obtained after cutting exactly passes through the engraved line 32a, the engraved line 32a can be seen on two side edges of the sensor, but is incomplete and imperfect, and it can be seen on one side of the sensor that the engraved lines 31b and 32b originally supposed to be distributed on two sides of the sensor have connected into one engraved line, at which point the cutter only cuts on the engraved line forming an electrode and the electrode itself is not damaged, that is, the electrode part at the sample contacting end of the sensor only has the engraved line being cut without damage to the electrode, however, the positions of the LOGO on the color layer of the sensor and the sample injection channel at the sample injection end have been out of the preset range of the central axis of the sensor, the performance of the sensor at this point is not affected, but the appearance thereof is unacceptable, so that the sensor can be considered to be a defective product. As shown in b in Fig. 15, it is a sensor obtained when large deviation occurs in the cutting process, the engraved lines for identifying cutting deviation appear completely only one side but do not appear on the other side, and it can be seen on one side of the sensor that the engraved lines 31b and 32b originally supposed to be distributed on two sides of the sensor have connected into one engraved line, at which point the LOGO on the color layer of the sensor and the sample injection groove at the sample injection end have completely deviated from the preset range of the central axis of the sensor, meanwhile the electrodes at the sample contacting end are also damaged, and the sensor at this point may be considered to be a scrapped sensor.

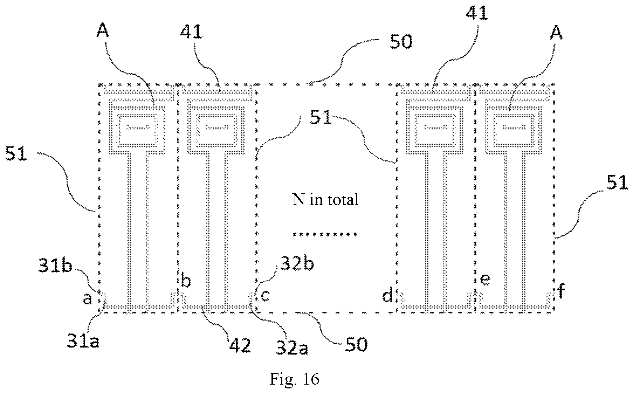

As shown in Fig. 16, it is a semi-finished large card with N sensor units A arranged on the conductive layer in the solution of this example subjected to laser engraving, the π-shaped engraved line on the sensor unit A consists of engraved lines 31b, 31a, 42, 32a and 32b, and the engraved lines 31b and 32b are connected between two adjacent sensor base units A, so that all π-shaped engraved lines for identifying cutting deviation at the contact ends of the N sensor base units A can be connected end to end to form a continuous engraved line. As shown in Fig. 16, when the laser etching technology is used to cut the conductive layer to generate the engraved lines 31b, 31a, 42, 32a and 32b, the laser engraving method can be performed in a manner that, instead of sequentially engraving the N sensor base units A, the engraved lines 31b, 31a, 42, 32a and 32b for identifying cutting deviation arranged in the same row on the conductive layer are consecutively cut from point a as a starting point to point f as a terminal point via points b, c, d, and e. That is, the engraved lines for identifying cutting deviation can be formed on N sensor base units A through once consecutive cutting. The design of Fig. 16 can effectively reduce and even avoid jumping of the laser path and idleness of laser, thereby increasing the production efficiency and enhancing the production capacity.

As shown in Fig. 11, since neither of the engraved lines 31a and 32a for identifying cutting deviation in the solution shown in Fig. 11 intersects the side edges of the sensor, jumping and idleness exist inevitably on the path of the laser between adjacent sensor units A. For example, after the engraved lines 31a, 42 and 32a of the first sensor base unit A are engraved, the laser will jump and will be idle and standby, and etching of the engraved lines 31a, 42 and 32a will be started until the laser comes to the position of the second sensor base unit A.

The present invention only takes the end-to-end connection of the engraved lines for identifying cutting deviation at the contact end as an example to introduce the help of this method to improve the engraving efficiency and the production capacity, but this solution is also applicable to all other engraved lines, and developers can achieve the end-to-end connection of the laser path simply through reasonable arrangement of the engraved lines and design of the laser path, which similarly can improve the efficiency and production capacity of the laser engraving.

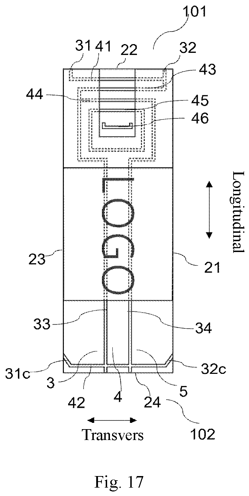

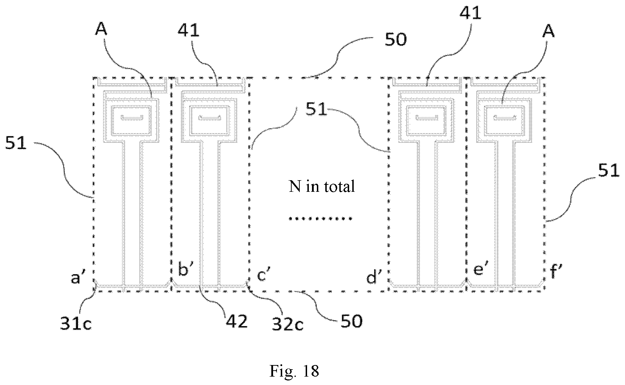

For the sensor as shown in Fig. 17, the engraved lines covered under the interlayer and the color layer are represented by dotted lines, the engraved line 42 is designed at the contact end of the sensor, and the engraved lines 31c, 32c for identifying cutting deviation are designed at the two side edges of the sensor. One end of each of the engraved lines 31c, 32c for identifying cutting deviation intersects the engraved line 42, and the other end intersects the side edges 23, 21 of the sensor. Each of the engraved lines 31c, 32c is at an obtuse angle to the engraved line 42, and when the complete engraved lines 31c and 32c for identifying cutting deviation as well as the obtuse angle formed by each of them with the engraved line 42 can be seen on both sides of the sensor, the sensor can be considered to be a qualified sensor. At the same time, this design can also ensure that the engraving of the N sensor units A on the conductive layer of the sensor can achieve the end-to-end connection of the engraved lines, specifically as shown in Fig. 18. **In** this figure, the interlayer and the color layer are omitted, and the etching of the engraved lines 31c, 32c and 42 in the N sensor units A on the conductive layer can be done starting from a' of the first sensor base unit A as a starting point, via b', c', d', e' of the sensor base unit A and finally ending at f of the Nth sensor base unit A, thereby achieving the end-to-end connection of the engraved lines, and similarly improving the efficiency and capacity of the laser engraving.

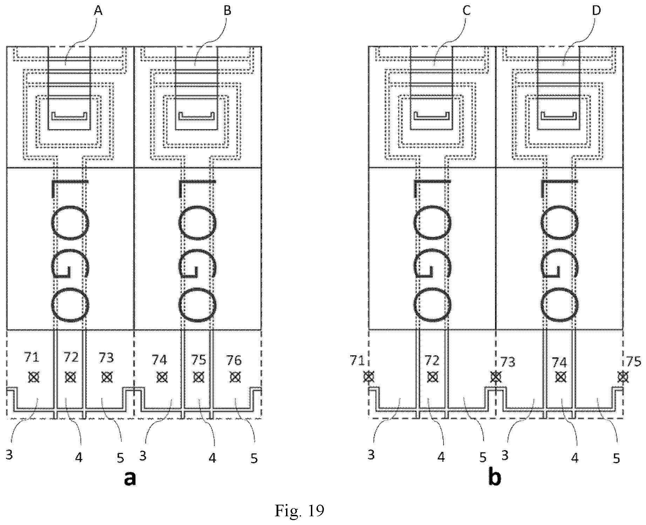

The solution shown in the present invention also has advantages over the prior art in terms of the specific production process. To ensure the performance of the produced product, quality inspection may be required at each step of the production process of the biosensor. More specifically, for a biosensor with electrodes formed by laser etching, the electrodes or conductive regions on the two sides separated by the same engraved line must be non-conductive to each other or insulated from each other, which thus needs to be confirmed and verified by using electrical means. Fig. 19 shows an uncut semi-finished product containing biosensors arranged side by side.

The insulated quality inspection sites on the semi-finished product can be disposed at six sites 71-76 as shown in a in Fig. 19, the quality inspection sites 71, 72, 73 are located on the electrodes 3, 4 and 5 of sensor A, and the quality inspection sites 74, 75, 76 are located on the electrodes 3, 4 and 5 of sensor B. The insulation quality inspection sites may also be disposed at five sites 71-75 as shown in b in Fig. 19, the quality inspection sites 71, 72 are located on the electrodes 3 and 4 of sensor C, respectively, the quality inspection sites 74, 75 are located on the electrodes 4 and 5 of sensor D, respectively, and the quality inspection site 73 is disposed in the connecting region of the electrode 5 of sensor C and the electrode 3 of sensor D, thus the electrode 5 of sensor C and the electrode 3 of sensor D can share one quality inspection site 73. Before cutting with the cutter, sensor A and sensor B are connected, and sensor C and sensor D are connected. In inspection of semi-finished products, the inspection sites may be in the manner of a in Fig. 19 or in the manner of b in Fig. 19 according to needs, and the number of quality inspection sites in b in Fig. 19 is less than that in a in Fig. 19.

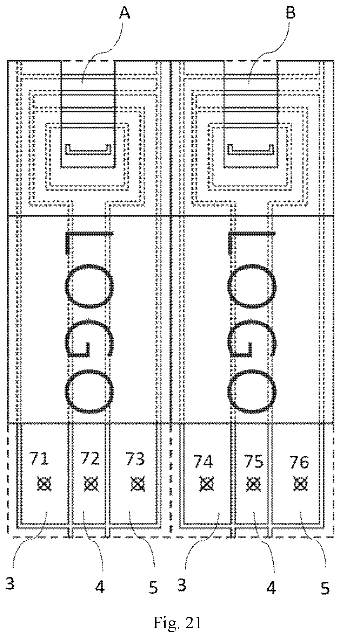

In the design solution shown in Fig. 21, the quality inspection sites 71, 72, 73 are located on the electrodes 3, 4 and 5 of sensor A, respectively, the quality inspection sites 74, 75, 76 are located on electrodes 3, 4 and 5 of sensor B, and sensor A and sensor B are connected before they are cut with the cutter. The electrode 5 of sensor A and the electrode 3 of sensor B adjacent thereto cannot share one inspection site. Thus, compared to the engraved line design in Fig. 19, more quality inspection sites are required in Fig. 21 to inspect the on/off conditions of the electrodes.

In the case where the semi-finished large cards as shown in Fig. 16 and Fig. 19 are cut into independent biosensors, when two or more biosensors are arranged side by side, the engraved lines 42, 31a, 31b, 32a, 32b of two biosensors located at the contact end can be connected to each other. For example, when two biosensors are arranged side by side, the engraved line 32b of the biosensor located on the left can be abutted to the engraved line 31b of the biosensor located on the right, so that when two biosensors are arranged side by side, the engraved lines 42, 31a, 31b, 32a, 32b, etc., of two adjacent biosensors can be connected to each other. Similarly, for the biosensors as shown in Figs. 17 and 18, when two or more biosensors are arranged side by side, the engraved lines 42, 31c, 32c for identifying cutting deviation located at the contact end can be connected together by abutment of the engraved line 32c of the biosensor located on the left with the engraved line 31c of the biosensor located on the right, thereby connecting these engraved lines together. When two or more biosensors are arranged side by side, engraved lines that can be connected to each other together are formed at the contact ends. In the example shown in Figs. 16 and 18, these interconnected engraved lines form repeating units of the same shape.

The following principle may be used to set the position of the engraved line (e.g. 42, 31a, 31b, 32a or 32b) for identifying cutting deviation as described in the present invention on the conductive layer. When the electrodes at the sample contacting end of the biosensor obtained after cutting by the cutter are not damaged due to the cutting, a more preferred way also includes that from the appearance of the biosensor obtained after cutting, the central axes of the color layer with the LOGO and the sample injection groove at the sample contacting end are both located on the central axis of the biosensor or do not deviate too much, so that the sensor has a better appearance. Specifically, the arrangement is for example, but not limited to the following ways.

Arrangement way 1: the distances from each of the two endpoints of the transverse engraved line 42 for identifying cutting deviation to the side edges 21 and 23 of the sensor are the same.

Arrangement way 2: if the distance between an endpoint of the engraved line 42 for identifying cutting deviation and a side edge of the sensor is L1, and the distance between the engraved line on the same side as the endpoint, for forming an electrode and closest to the side edge of the sensor, and the side edge of the sensor is L2, L1 is less than or equal to L2.

Arrangement way 3: the engraved lines forming electrodes include transverse engraved lines and vertical engraved lines, and the engraved lines close to the edge of the conductive layer are outer engraved lines, and the engraved lines away from the same edge line of the conductive layer are inner engraved lines. If the distance between an endpoint of the engraved line for identifying cutting deviation and an edge line of the conductive layer is L1, and the closest distance between an outer vertical engraved line of the electrode that is closest to the same edge of the conductive layer and the edge of the conductive layer is L3, L1 is less than or equal to L3.

### Example 4: glucose test data

The present invention will be further illustrated below in conjunction with the biosensor corresponding to Fig. 14 of the solution in Example 3.

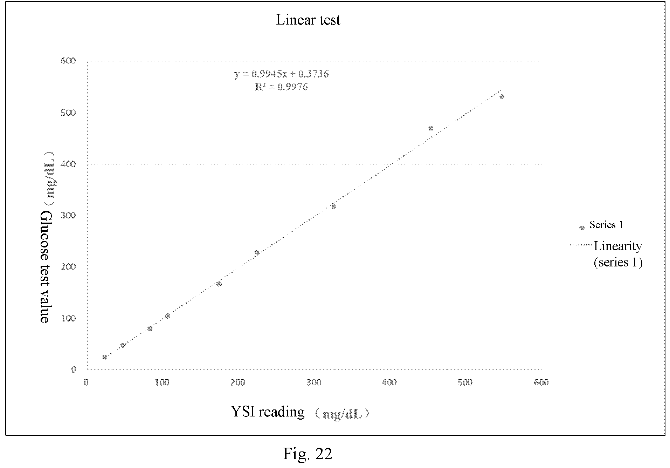

Fig. 22 shows an exploded oblique view of the biosensor corresponding to Fig. 14 of Example 3. The glucose biosensor in this example includes an insulating substrate 1, a conductive carbon layer 2, a reagent layer 6, an interlayer 8 and an upper cover 9. Specifically, the glucose biosensor of this example includes a conductive layer 2 of uniform thickness, which is prepared by a coating process and located on the insulating substrate 1. Laser engraved lines 31a, 31b, 32a, 32b, 33, 34, 41, 42, 43, 44, 45, 46 and the like are etched on the conductive carbon coating using the laser etching technology according to a reasonable programming design, and the conductive layer is removed by ablation of the laser engraved lines to expose the insulating substrate, thus forming electrodes 3, 4, 5 on the conductive layer 2 that are not connected to each other. The reagent layer 6 is located on the electrodes 3, 4, 5 in a sample injection groove 81 of the interlayer 8. The interlayer 8 is covered with the upper cover 9, and the air hole 91 in the upper cover is located at the bottom of the sample injection groove 81 of the interlayer. The sample injection groove 81 of the interlayer, and the hydrophilic upper cover 9 and the air hole 91 above the sample injection groove form a blood sample injection channel. The color layer 7 is printed with LOGO or color or the like to distinguish the sensor.

After the glucose sensor is inserted into the test instrument, the test instrument is started, the blood sample is sucked into the sample injection channel by siphoning, and the air in the original sample injection channel is discharged through the air hole 91 in the upper cover at the tail end of the sample injection channel, which effectively ensures the smoothness that the blood flows into the channel. When the blood enters to fill in the channel, a DC voltage of 200-500mv is applied to the electrode so that the reagent layer 6 carries out a redox reaction with the glucose to be tested in the blood and generates a test current. The test current is appropriately corrected and compensated for by the test instrument according to the detected current value and the ambient temperature measured by a temperature sensor, and is converted into a glucose value for display to the user.

The materials of the reagent layer 6 mainly include glucose dehydrogenase FAD-GDH, potassium ferricyanide and a second electron enzyme mediator; and the enzyme activity of the glucose dehydrogenase is within the range of 200-600U/mg. 11 blood samples with different glucose concentrations are tested at room temperature using the above glucose sensor. The hematocrits of different concentrations of blood samples are adjusted to 42% ± 2% before the test, and their specific concentrations are obtained through testing with a glucose lactate analyzer (model YSI 2300 STAT) manufactured by YSI Corporation, U.S.A. The test is repeated 10 times for each concentration of blood sample, and the test results are shown in Table 1.

**Table 1 Test results of glucose sensor**

| YSI reading (mg/dl) | 11 | 23 | 47 | 83 | 106 | 174 | 224 | 325 | 453 | 547 | 64 7 |
|---|---|---|---|---|---|---|---|---|---|---|---|
| Reading 1 | L O | 26 | 49 | 83 | 111 | 163 | 232 | 318 | 479 | 544 | HI |
| Reading 2 | L O | 25 | 50 | 79 | 103 | 164 | 236 | 315 | 478 | 537 | HI |
| Reading 3 | L O | 25 | 50 | 81 | 108 | 174 | 230 | 323 | 477 | 530 | HI |
| Reading 4 | L O | 25 | 47 | 80 | 110 | 161 | 230 | 318 | 473 | 541 | HI |
| Reading 5 | L O | 25 | 47 | 87 | 107 | 167 | 225 | 327 | 482 | 510 | HI |
| Reading 6 | L O | 25 | 48 | 77 | 104 | 165 | 223 | 312 | 471 | 520 | HI |
| Reading 7 | L O | 24 | 47 | 82 | 104 | 171 | 224 | 309 | 471 | 525 | HI |
| Reading 8 | L O | 24 | 48 | 81 | 99 | 167 | 223 | 317 | 456 | 548 | HI |
| Reading 9 | L O | 22 | 45 | 80 | 105 | 167 | 223 | 331 | 447 | 532 | HI |
| Reading 10 | L O | 23 | 45 | 78 | 105 | 172 | 248 | 310 | 464 | 525 | HI |
| Average value | --- - | 24.4 | 47.6 | 80.8 | 105. 6 | 167. 1 | 229. 4 | 318. 0 | 469. 8 | 531. 2 | ---- |
| Standar d deviatio n | --- - | 1.17 | 1.78 | 2.82 | 3.53 | 4.15 | 7.95 | 7.20 | 11.1 0 | 11.69 | ---- |
| Test precisio n | --- - | 4.8 % | 3.7 % | 3.5 % | 3.3% | 2.5% | 3.5 % | 2.3% | 2.4 % | 2.2% | ---- |
| Test deviatio n | --- - | 1.5 | 0.2 | -1.8 | -0.3 % | -4.0 % | 2.4 % | -2.2 % | 3.8 % | -2.8 % | ---- |

It can be seen from the comparison of the average of 10 repeated tests of blood at each glucose concentration and the test results of YSI 2300 that in the range of very low glucose concentration (less than 20mg/dl) and in the range of very high glucose concentration (more than 600mg/dl), the test results of the instrument show LO and HI, respectively. Except for at these extreme concentrations, the glucose sensor has very small test deviation and high accuracy. In addition, the coefficients of variation of the test values of the same blood sample are all within 3%, and the precision completely meets requirements.

Fig. 22 is a line graph of the glucose sensor test values versus the YSI test values of the example, with a fitting equation y = 0.9945x + 0.3736; and R² = 0.9976. It can be seen that the glucose sensor has a good line shape.

## Claims

1. A biosensor, comprising an insulating substrate, a sample injection port, a fluid channel for entrance of a test sample, and a conductive layer disposed on the insulating substrate, engraved lines and electrodes divided by the engraved lines being distributed on the conductive layer, a reagent layer being disposed on part or all of the electrodes located in the region of the fluid channel, wherein a transverse engraved line (42) is disposed at a contact end on the other side opposite to the sample injection port of the biosensor, and at least one end of the transverse engraved line (42) extends towards, but does not intersect with a longitudinal side edge of the biosensor.

2. The biosensor according to claim 1, wherein no engraved line exists between the end of the transverse engraved line (42) extending towards the longitudinal side edge of the biosensor and the longitudinal side edge.

3. The biosensor according to claim 1, wherein the end of the transverse engraved line (42) close to the longitudinal side edge of the biosensor is connected with a vertical engraved line (31a or 32a), and the vertical engraved line is parallel to or at a certain angle to the longitudinal side edge of the biosensor.

4. The biosensor according to claim 3, wherein no engraved line exists between the vertical engraved line (31a or 32a) and the longitudinal side edge of the biosensor on the same side.

5. The biosensor according to claim 1, wherein two ends of the transverse engraved line (42) extend towards, but do not intersect with two longitudinal side edges of the biosensor.

6. The biosensor according to claim 5, wherein two ends of the transverse engraved line (42) are connected with a vertical engraved line (31a or 32a), and the vertical engraved line is parallel to or at a certain angle to the longitudinal side edge of the biosensor.

7. The biosensor according to claim 6, wherein the vertical engraved line (31a or 32a) intersects a side edge of the biosensor.

8. The biosensor according to claim 6, wherein the other end of the vertical engraved line (31a or 32a) opposite to the end connected with the transverse engraved line (42) is also connected with another transverse engraved line (31b or 32b), and the other transverse engraved line (31b or 32b) intersects the side edge of the biosensor.

9. The biosensor according to claim 8, wherein the transverse engraved line (42), the vertical engraved line (31a or 32a) and the other transverse engraved line (31b or 32b) are connected to be ₇r-shaped.

10. The biosensor according to claim 6 or 9, wherein when two or more biosensors are arranged together side by side, adjacent engraved lines of two adjacent biosensors can be connected to each other.

11. The biosensor according to claim 10, wherein when two or more biosensors are arranged together side by side, adjacent vertical engraved lines of two adjacent biosensors can be connected to each other, or adjacent other transverse engraved lines of the two adjacent biosensors can be connected to each other.

12. The biosensor according to claim 1, wherein the end of the transverse engraved line (42) extending towards the longitudinal side edge of the biosensor is observable.

13. The biosensor according to claim 8, wherein the transverse engraved line, the vertical engraved line and the other transverse engraved line are observable.

14. The biosensor according to claim 1, wherein the transverse engraved line located at the contact end stretches across the electrodes or the region that the electrodes extend towards the contact end.

15. The biosensor according to claim 1, wherein the biosensor comprises a working electrode and a counter electrode.

16. The biosensor according to any of claims 1 to 15, wherein the transverse engraved line or the vertical engraved line is set in such a way that when the transverse engraved line or the vertical engraved line on the biosensor meets preset requirements, the electrode at the sample contacting end of the biosensor is complete, and/or the central axis of a sample injection groove is located on the central axis of the biosensor or deviates to an extent within a preset range.

17. A method for determining whether a biosensor is cut correctly when being manufactured, comprising: setting an engraved line for identifying cutting deviation at a contact end on the other side of the biosensor opposite to the sample injection port, the engraved line for identifying cutting deviation comprising a transverse engraved line, at least one end of the transverse engraved line extending towards, but does not intersecting with a longitudinal side edge of the biosensor; and observing whether the engraved line for identifying cutting deviation meets pre-designed requirements, and if not, determining that the cutting is incorrect.

18. The biosensor according to claim 17, wherein the engraved lines of the biosensor are according to any one of claims 1 to 15, the finished biosensor obtained after cutting is observed, and if a biosensor does not completely render the engraved lines according to any one of claims 1 to 15, it is determined that the cutting is incorrect.

19. A method for preparing a biosensor, comprising the following steps: etching engraved lines and electrodes divided by the engraved lines on an insulating substrate having a conductive layer according to a pre-designed electrode pattern, and etching the engraved lines according to any one of claims 1 to 15 to form a large electrode card having a plurality of sensor base units; adding a prepared reagent to corresponding electrodes requiring addition of the reagent; affixing an interlayer to the sample contacting end of each sensor base unit; providing an upper cover above the interlayer to obtain a semi-finished large card; cutting the semi-finished large card by using a cutter along a preset cutting line to obtain finished biosensors; and removing the biosensors that do not completely render the engraved lines according to any one of claims 1 to 15 and are unqualifiedly cut, whereas retaining the biosensors that are qualifiedly cut.
